Europäisches Patentamt

European Patent Office

Office européen des brevets

(11) Veröffentlichungsnummer: **0 377 429**

**A1**

(12) # EUROPÄISCHE PATENTANMELDUNG

(21) Anmeldenummer: **89890321.6**

(22) Anmeldetag: **15.12.89**

(51) Int. Cl.⁵: **C12M 1/04, B01J 10/00, C02F 3/12**

(30) Priorität: **21.12.88 AT 3128/88**

(43) Veröffentlichungstag der Anmeldung:
**11.07.90 Patentblatt 90/28**

(84) Benannte Vertragsstaaten:
**BE CH DE ES FR GB GR IT LI LU NL SE**

(71) Anmelder: **Maschinenfabrik Andritz
Actiengesellschaft
Statteggerstrasse 18
A-8045 Graz-Andritz(AT)**

(72) Erfinder: **Paar, Heinz, Dr.
Berg 5
A-9073 Klagenfurt(AT)**
Erfinder: **Nussbaumer, Manfred, Dr.
Herrengasse 30
A-4820 Bad Ischl(AT)**
Erfinder: **Moser, Anton, Prof. Dr.
Im Hoffeld 24
A-8045 Graz(AT)**

(74) Vertreter: **Collin, Hans, Dipl.-Ing. Dr.
Patentanwälte Dipl.-Ing. Dr. Hans Collin
Dipl.-Ing. Erwin Buresch Dipl.-Ing.Armin
Häupl Mariahilferstrasse 50
A-1070 Wien(AT)**

(54) **Verfahren zum kontinuierlichen Durchführen von Reaktionen sowie Rohrreaktor zur Durchführung des Verfahrens.**

(57) Vorgeschlagen wird ein Verfahren zum kontinuierlichen Durchführen von Reaktionen, bei dem ein flüssiges Medium, vorzugsweise als Pfropfenströmung, unter Begasung und/oder Belüftung von unten, durch einen Horizontalrohrreaktor hindurchgefördert wird, mit dem Kennzeichen, daß im flüssigen Medium eine sich axial im Rohrreaktor erstreckende, durchgehende Blasenwand aus Belüftungs- und/oder Begasungsmedium gebildet wird, sowie ein Rohrreaktor zu seiner Durchführung, mit axial verlaufenden Begasungs- und/oder Belüftungsöffnungen am Reaktorboden, bei dem die Begasungs- und/oder Belüftungsöffnungen mit Bezug aufeinander so benachbart, überläppt und/oder versetzt angeordnet sind, daß das aus ihnen austretende Begasungs- und/oder Belüftungsmedium im im Reaktor strömenden flüssigen Medium eine aufsteigende, durchgehende Blasenwand bildet.

Fig. 1

## Verfahren zum kontinuierlichen Durchführen von Reaktionen sowie Rohrreaktor zur Durchführung des Verfahrens

Die Erfindung betrifft ein Verfahren zum kontinuierlichen Durchführen von Reaktionen, bei dem ein flüssiges Medium, vorzugsweise als Pfropfenströmung, unter Begasung und/oder Belüftung von unten, durch einen Horizontalrohrreaktor hindurchgefördert wird, sowie einen Rohrreaktor mit axial verlaufenden Begasungs- und/oder Belüftungsöffnungen zu seiner Durchführung.

Rohrreaktoren, auch Horizontalrohrreaktoren, für flüssige Medien sind bekannt und werden industriell auf verschiedenen Gebieten, z.B. in der Milchindustrie, der Abwasseraufbereitung und der biologischen Reaktionstechnologie, eingesetzt. Sie stellen die für eine kontinuierliche Fahrweise, besonders bei biologischen Reaktionen, am besten geeignete Reaktorform dar.

Hiebei ist auch schon festgestellt worden, daß die günstigste Strömungsform in einem Rohrreaktor eine sogenannte Pfropfenströmung wäre, d.h. eine Strömung ohne jede Rückvermischung. Dieser Idealfall ist in der biotechnologischen Praxis selbst bei Reaktoren, die ihrem Habitus nach als Rohr ausgeformt sind, durch die langen Verweilzeiten und damit verursachten langsamen Strömungsgeschwindigkeiten nicht annähernd erreichbar, insbesondere, wenn das Fermentationsmedium begast und belüftet werden muß und somit eine zusätzliche Störung des Strömungsmusters eintritt. Annäherungen an das Ziel einer Pfropfenströmung sind z.B. in den AT-PSen 323 082 und 340 839 beschrieben, wobei hier eine an der untersten Erzeugenden des Rohres installierte und eigens ausgeformte Begasungsvorrichtung die Pfropfenströmung hervorruft. Dort wird angegeben, daß zur Erzielung einer Längsströmung ohne wesentliche Rückvermischung das Verhältnis von Rohrlänge zu hydraulischem Durchmesser nicht unter einem gewissen Mindestwert liegen sollte und daß man mit der Strömungsgeschwindigkeit, der Dimensionierung und/oder Anordnung der Belüftungsaggregate sowie dem Druck und der Menge des zugeführten Gases Regelgrößen zur Verfügung hat, um die angestrebte Strömungsform zu erzielen, wobei selbstverständlich die Begasung so erfolgen soll, daß eine möglichst geringe Rückvermischung auftritt. Als Mittel dazu sind in der AT-PS 323 082 Lochdüsen und Strahldüsen angegeben, die längs einer Erzeugenden des Reaktorrohrs angeordnet sind.

Es wurde nunmehr gefunden, daß sich mit dieser bekannten Konstruktion mit in Abständen voneinander angeordneten Düsen die Rückvermischung mit zunehmender Begasung, die im Falle aerob zu betreibender Prozesse mit geforderten Mindestsauerstoffeintragswerten notwendig sein kann, erhöht und somit die angestrebte Strömungscharakteristik, durch die verstärkte Ausbildung axialer Strömungswalzen, verschlechtert.

Da die Gasdüsen bei den bekannten Anordnungen in axialem Abstand voneinander angeordnet sind, ist - ruhende Flüssigkeit vorausgesetzt - jeder Gasdüse somit an beiden Seiten ein axialer Kreislauf, Zirkulationswalze genannt, zugeordnet, so daß zwischen je zwei benachbarten Gasdüsen eine Rückvermischungszone auftritt.

Zur Vermeidung dieses Nachteils wird beim erfindungsgemäßen Verfahren vorgeschlagen, daß im flüssigen Medium eine sich axial im Rohrreaktor erstreckende, durchgehende Blasenwand aus Belüftungs- und/oder Begasungsmedium gebildet wird.

Auf diese Weise wird die Ausbildung axialer Zirkulationswalzen weitestgehend unterdrückt, und die durch die Gasblasen nach oben mitgeführte Flüssigkeit gezwungen, über die radial entstehenden Strömungswalzen nach unten zu strömen. Durch die Ausbildung solcherart erwünschter rein radialer kreis- bzw. nierenförmiger Strömungswalzen im Rohrquerschnitt entstehen scheibenförmige Flüssigphasensegmente, die sich relativ stoffaustauschinert gegenüber ihren Nachbarelementen verhalten und somit eine geringe Rückmischungstendenz aufweisen.

Dementsprechend ist der erfindungsgemäße Horizontalrohrreaktor zur Durchführung dieses erfindungsgemäßen Verfahrens vor allem dadurch gekennzeichnet, daß die Begasungs- und/oder Belüftungsöffnungen mit Bezug aufeinander so benachbart, überlappt und/oder versetzt angeordnet sind, daß das aus ihnen austretende Begasungsund/oder Belüftungsmedium im im Reaktor strömenden flüssigen Medium eine aufsteigende, durchgehende Blasenwand bildet.

Die Begasungs- und/oder Belüftungsöffnungen sind vorteilhaft an einer vorzugsweise ebenflächigen Membran ausgebildet. Als Membran sind hier z.B. Körper mit durchgehenden Poren, z.B. aus Kunststoff, Sintermetall oder Keramik, oder dünne Metallbleche mit feinen Löchern und/oder Schlitzen zu verstehen, die z.B. nach der Funkenerosionsoder Lasertechnologie angebracht werden können.

Es wird bevorzugt, daß diese Membranen nicht Teil der Reaktorrohrwandung sind, obwohl dies möglich ist (z.B. durch Einschweißen), sondern Teile eines in den Reaktor eingesetzten Rohrs oder Schlauchs sind oder diesen selbst bilden. Dies bewirkt eine schnelle Auswechselbarkeit und ist auch dort vorteilhafter, wo unter sterilen Bedingungen gefahren werden soll. Das Profil des Rohrs oder Schlauchs ist vorzugsweise rund oder elliptisch, kann aber z.B. auch rechteckig sein.

Vorzugsweise sind Begasungs- und/oder Belüftungsöffnungen mit geringem Durchmesser, insbesondere im Bereich von <10 μm, vorzugsweise von 3 bis 6 μm, vorgesehen, so daß beispielsweise Gasbläschen mit einer Maximalgröße von etwa 1 mm erzielbar sind.

Der erfindungsgemäße Rohrreaktor ist weiterhin vorzugsweise so ausgebildet, daß das Verhältnis Reaktorrohrlänge zu hydraulischem Durchmesser mindestens 10:1 beträgt. Dabei ist der hydraulische Durchmesser $d_h$ definitionsgemäß gleich $\frac{4F}{U}$, worin F den benetzten Querschnitt und U den dazugehörigen Umfang bedeutet. Durch diese schlanke Reaktorform läßt sich über einen großen Bereich von Strömungsgeschwindigkeiten eine gute Pfropfenströmung erzielen.

Ein weiterhin bevorzugtes Mittel zur Annäherung an eine ideale Pfropfenströmung ist beim erfindungsgemäßen Reaktor dadurch gegeben, daß im Abstand voneinander angeordnete, zumindest im wesentlichen im rechten Winkel zur Reaktorrohrachse verlaufende Einbauwände vorgesehen sind, wobei vorzugsweise der Abstand zwischen den Einbauwänden etwa dem Reaktorrohrinnendurchmesser entspricht. Durch diese Anordnung von lamellenartigen Einbauwänden, die den freien Durchtrittsquerschnitt im Reaktorrohr verkleinern, kann die Bodensteinzahl, deren Wert umso höher wird, je mehr man sich der idealen Pfropfenströmung nähert, beträchtlich erhöht werden. Die Bodensteinzahl Bo ist definitionsgemäß

$$Bo = \frac{u \cdot L}{D_{ax}},$$

worin μ die mittlere Flüssigphasengeschwindigkeit, L die Reaktorrohrlänge und $D_{ax}$ der axiale Dispersionskoeffizient ist.

Bevorzugt ist es, wenn die Einbauwände bei kreisförmigem Reaktorrohrquerschnitt die Form eines Kreisabschnitts aufweisen und im unteren Bereich des Rohrquerschnitts an der Rohrwand anliegen, so daß zwischen Reaktorrohrwand und Einbauwand jeweils ein sichelförmiger Spalt gebildet ist.

Insbesondere dann, wenn die Begasung und/oder Belüftung über ein(en) am Boden des Reaktorrohrs eingesetzten Schlauch oder Rohr erfolgt, weisen die Einbauwände vorteilhaft im unteren Bereich einen, vorzugsweise keilförmigen, Ausschnitt auf.

Vorteilhaft sind die Einbauwände an einem in das Reaktorrohr einschiebbaren Träger befestigt, so daß u.a. eine rasche Umstellung auf eine geänderte Betriebsweise des Reaktors möglich ist (z.B.

Verwendung der Einbauten als Träger eines biologischen Rasens oder trägerfixierter Zellen und/oder Enzyme).

Für die Durchführung von Reaktionen, die eine Temperaturführung erfordern, ist vorteilhaft vorgesehen, daß das Reaktorrohr in bekannter Weise als Mantelrohr aufgebaut ist, wobei der Mantelinnenraum vorzugsweise in Abschnitte unterteilt ist, die eine gezielte Temperaturführung über die Rohrlänge zulassen.

Dabei muß natürlich kein über die Reaktorrohrlänge durchgehender Mantel vorgesehen sein; es ist auch möglich, mehrere Mäntel in axialem Abstand voneinander nach Wunsch anzubringen.

Für die Durchführung von z.B. strenge Sterilität erfordernden Reaktionen kann es günstig sein, ein einstückiges Reaktorrohr vorzusehen; bevorzugt ist jedoch, daß das Reaktorrohr aus aneinandergeflanschten Abschnitten gebildet ist, so daß der Reaktor nach dem Baukastensystem an die gewünschten Verhältnisse anpaßbar ist.

Vorteilhaft sind weiterhin in Abständen über die Reaktorrohrlänge verteilte Abfuhr- und/oder Zufuhrstutzen für Medium und/oder Kontrollstutzen vorgesehen, so daß eine weitere Steuerungsmöglichkeit für den Reaktionsablauf gegeben ist. Dies betrifft insbesondere eine dadurch mögliche Gradientenbildung in der Substratzusammensetzung (z.B. pH-Wert, $pO_2$-Konzentration) und die gesteuerte Zugabe von Reaktionspartnern oder -beeinflussern (Promotoren, Katalysatoren, Blockern etc.) zum gewünschten Zeitpunkt.

Das erfindungsgemäße Verfahren samt Vorrichtung ist vor allem gedacht für die kontinuierliche Durchführung biokatalysierter Reaktionen mit autokatalytischem Verhalten, z.B. Vergärung von Glukose zu Alkohol mit Zymomonas mobilis, und für den Einsatz von natürlichen und rekombinanten Mikroorganismen, Enzymen, Kulturen von pflanzlichen und tierischen Zellen, r DNA-Produkten u.dgl.

Dabei können z.B. die Mikroorganismen dem Substrat kontinuierlich zugegeben und nachher wieder entfernt werden, z.B. durch mechanische oder 2-Phasentrennung; ein Teil der gebildeten Mikroorganismen kann ins Verfahren rückgeführt werden. Oder es können trägerfixierte Mikroorganismen eingesetzt werden, wobei die Mikroorganismen auf Rohreinschüben fixiert sind, die vom Substrat durchströmt werden.

Ähnliches gilt für trägerfixierte Enzyme, wobei körnige Träger im Substrat transportiert werden (Gleich- oder Gegenstrom) oder die Enzyme auf Rohreinschüben fixiert sind.

Das erfindungsgemäße Verfahren samt erfindungsgemäßem Rohrreaktor wird im folgenden an Hand eines Ausführungsbeispiels unter Bezugnahme auf die Zeichnung näher beschrieben, in der Fig. 1 schematisch eine Fermentationsanlage und die Figuren 2 und 3 Details eines Reaktorrohreinschubs mit Lamelleneinbauwänden zeigen.

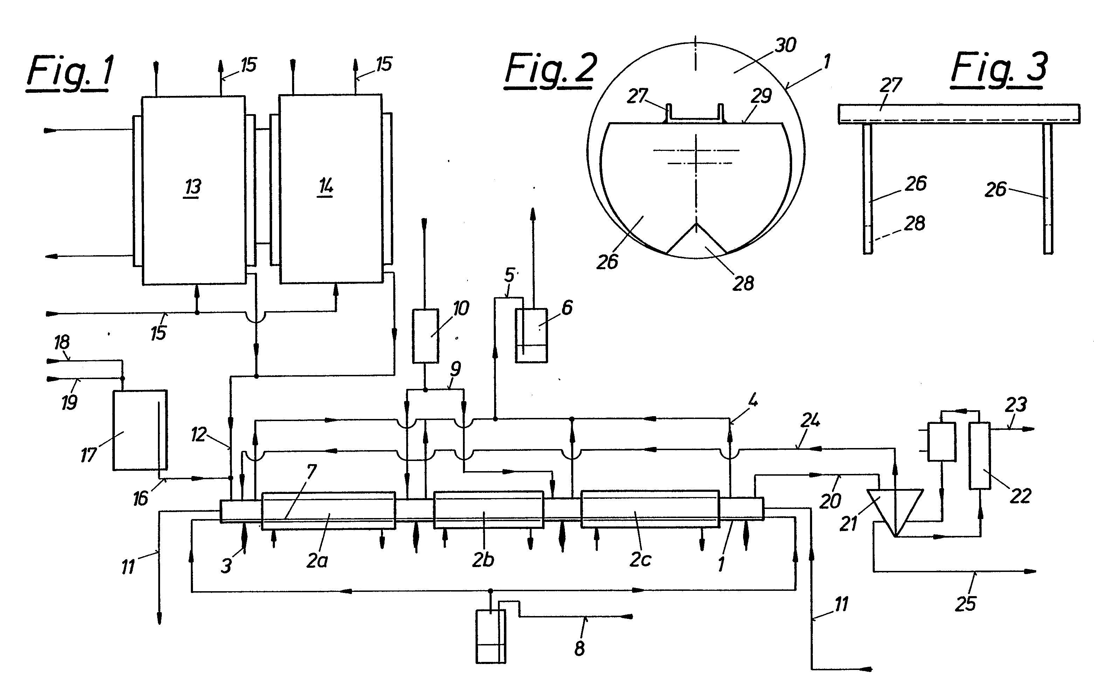

In Fig. 1 ist ein Bioreaktorsystem mit einem horizontalen Reaktorrohr 1 mit Kreisquerschnitt dargestellt, das durch drei Heizund/oder Kühlmäntel 2a,2b,2c in drei von außen beeinflußbare Reaktionszonen gegliedert ist. Am Anfang und am Ende des Reaktorrohrs 1 sowie zwischen den Reaktionszonen befinden sich ein oder mehrere schematisch angedeutete Anzapf- bzw. Zuführstutzen 3, die u.a. zur Probenahme, Einführen von Meßsonden und sonstiger Prozeßkontrolle dienen. An den angegebenen Stellen befinden sich im Oberteil des Reaktorrohrs 1 Gasableitungen 4 zur Aufnahme von abzuleitenden Prozeßgasen, die zu einer Gasableitung 5 zusammengefaßt sind, die ihrerseits über ein Fallengefäß 6 (z.B. Schaumfalle) in die Atmosphäre mündet. Am Boden des Reaktorrohrs 1 ist entlang der untersten Rohrerzeugenden ein Begasungs- bzw. Belüftungsrohr 7 in das Reaktorrohr 1 eingelegt. Das Begasungs- und/oder Belüftungsrohr 7 ist im betrachteten Beispiel ein Polyäthylenschlauch mit einer durchgehenden Porosität von etwa 50 % und Porendurchmessern zwischen 3 und 6 μm, der von beiden Enden des Reaktorrohrs 1 her über eine Gaszuführleitung 8, in der wiederum ein Fallengefäß 9 angeordnet ist, begast wird, wobei bei einem Leitungsdruck von 1,1 bar in dem im Reaktorrohr 1 strömenden flüssigen Medium Gasbläschen mit einem Durchmesser von etwa 1 mm erzielt werden. Die Wahl des zugeführten Gases hängt vom ablaufenden Prozeß ab und ist z.B. Stickstoff und/oder anderes Inertgas bei anaeroben, und sauerstoffhaltige Gase, insbesondere Luft, bei aeroben Prozessen.

Zwischen den Reaktionszonen 2a und 2b ist jeweils eine Zuführleitung 9 zum Eindosieren von Reaktionsbeeinflussern in das Reaktorrohr 1 zu erkennen, die unabhängig voneinander betrieben und über den Vorratsbehälter 10 gespeist werden. Selbstverständlich kann auch an anderen Stellen, z.B. bereits am Beginn des Reaktorrohrs 1, ein der mehrere Reaktionsbeeinflusser eindosiert werden.

Weiterhin weist das Reaktorrohr 1 eine Spülleitung 11 auf, mittels der das Innere des Reaktorrohrs 1 durchgespült werden kann, beispielsweise zur Sterilisation mit Dampf, Äthylenoxid od.dgl. Zur Einbringung des im Reaktorrohr 1 reagierenden flüssigen Substrats am Beginn des Reaktorrohrs ist eine Substratzuführleitung 12 vorgesehen, die über zwei Vorratstanks 13,14, die beispielsweise in Taktverfahren angezapft werden, gespeist werden. Die Vorratstanks weisen Heiz- und/oder Kühlmäntel auf sowie Zu- und Ableitungen 15 für Spülgas.

In die Substratzuführleitung 12 mündet eine weitere Zuführleitung 16, über die z.B. Mikroorganismen als Zellmasse oder auf körnigen Trägern fixierte Enzyme eindosierbar sind. In Fig. 1 ist ein Vorreaktor 17 eingezeichnet, der z.B. als Rührreaktor oder Airliftreaktor ausgebildet ist und zur Vorvermehrung von Mikroorganismen dient, der über eine Zuführleitung 18 mit Mikroorganismen beschickt wird und diese dann über die Leitung 16 in die Substratzuführleitung 12 in das Reaktorrohr 1 abgibt.

Wiederum ist der Vorreaktor auch mit einer Spülgaszuführleitung 19 versehen, z.B. um je nach Bedarf die im Vorreaktor 17 gewünschten Verhältnisse (Sterilisieren, aerobe oder anaerobe Atmosphäre) zu schaffen.

Wird das in Fig. 1 gezeigte Reaktorsystem zur anaeroben Vergärung von Glukose zu Äthylalkohol eingesetzt, ist das Substrat eine Glukoselösung; als Mikroorganismen werden eine Zellkultur von Zymomonas mobilis, vorzugsweise der unter ATCC 10988 hinterlegte Stamm, dem Reaktorrohr zugeführt. Das erzeugte Endprodukt wird über die Austragleitung 20 aus dem Reaktorrohr 1 ausgetragen und gelangt in den Ausgleichsbehälter 21 einer Trennvorrichtung 22 (Ultrafiltrationseinrichtung), wo die Zellmasse von der flüssigen Phase getrennt wird. Die flüssige Phase enthält das gewünschte Fermentationsprodukt und wird über den Auslaß 23 abgeführt; die angereicherte Zellmasse geht über die Rückführleitung 24 kontinuierlich zurück zum Substrateinspeisebereich des Reaktorrohrs 1. Wenn durch diese Zellmassekreisführung die Zellenkonzentration am Reaktoreingang den gewünschten Wert erreicht hat, wird die Zellmassezufuhr über die Leitung 16 abgebrochen; die Anfahrphase ist damit beendet und die eigentliche Produktionsphase beginnt, während der die Zellenkonzentration weiter ansteigen würde, wenn die gesamte nach dem Reaktorausgang abgetrennte Zellmasse rückgeführt würde. Um dies zu verhindern, ist eine Ausgleichsleitung 25 (bleed-Leitung) vorgesehen, über die die überschüssige Zellmasse dosiert aus dem System abführbar ist. Sie wird in der Regel verworfen.

Das in Fig. 1 dargestellte System ist bezüglich aller Verfahrensparameter genau steuerbar, die entsprechende Ausrüstung gehört zum bekannten Stand der Technik. Die Anlage kann ohne Schwierigkeiten steril gefahren werden.

Die Fig. 2 und 3 zeigen die Anordnung von im rechten Winkel zur Achse des Reaktorrohrs 1 verlaufenden Einbauwänden 26, die auf einem Träger 27 in Form eines U-Profils etwa in einem dem Reaktorrohrdurchmesser entsprechenden Abstand angeordnet sind. Der Träger 27 mit den Einbauwänden 26 bildet eine Einschubeinheit, die so in das Reaktorrohr 1 eingesetzt wird, daß sie unten am Reaktorrohr anliegt. Das Profil der Einbauwände 26 ist das einer ab- bzw. ausgeschnittenen Kreisscheibe, deren Durchmesser etwa 85 % des Innendurchmessers des Reaktorrohrs 1 ist. Jede Einbauwand 26 hat unten einen keilförmigen Ausschnitt 28, um Platz für das in diesem Bereich angeordnete Begasungs- und/oder Belüftungsrohr 7 zu lassen.

Der Substratspiegel im Reaktorrohr 1 wird (geringfügig) unterhalb der Oberkante 29 der Einbauwände 26 gehalten, so daß der Flüssigkeitstransport im wesentlichen nur in den unteren Seitenbereichen des zwischen Reaktorrohrinnenwand und den Einbauwänden verbleibenden, im Profil mondsichelförmigen Zwischenraum 30 stattfindet.

Die in den Fig. 2 und 3 gezeigte Ausführungsform ist für einen Substratpegel von etwa der Hälfte bis zu etwa zwei Drittel des Reaktorrohrinnendurchmessers vorgesehen.

Um ein möglichst gleichmäßiges Pfropfenströmungsprofil zu erzielen, ist am günstigsten ein gerades Reaktorrohr; mit etwas schlechteren Ergebnissen können auch möglichst sanft gekrümmte Reaktorrohre verwendet werden, z.B. Schlaufenrohre, wobei es günstig ist, wenn auf eine Krümmung eine Gegenkrümmung folgt.

## Ansprüche

1. Verfahren zum kontinuierlichen Durchführen von Reaktionen, bei dem ein flüssiges Medium, vorzugsweise als Pfropfenströmung, unter Begasung und/oder Belüftung von unten, durch einen Horizontalrohrreaktor hindurchgefördert wird, dadurch gekennzeichnet, daß im flüssigen Medium eine sich axial im Rohrreaktor erstreckende, durchgehende Blasenwand aus Belüftungs- und bzw. oder Begasungsmedium gebildet wird.

2. Horizontalrohrreaktor zur Durchführung des Verfahrens nach Anspruch 1, mit axial verlaufenden Begasungs- und/oder Belüftungsöffnungen am Reaktorboden, dadurch gekennzeichnet, daß die Begasungs- und/oder Belüftungsöffnungen mit Bezug aufeinander so benachbart, überlappt und/oder versetzt angeordnet sind, daß das aus ihnen austretende Begasungs- und/oder Belüftungsmedium im im Reaktor strömenden flüssigen Medium eine aufsteigende, durchgehende Blasenwand bildet.

3. Reaktor nach Anspruch 2, dadurch gekennzeichnet, daß die Begasungs- und/oder Belüftungsöffnungen an einer vorzugsweise ebenflächigen Membran ausgebildet sind.

4. Reaktor nach Anspruch 2 oder 3, dadurch gekennzeichnet, daß die Begasungs- und/oder Belüftungsöffnungen an einem in den Reaktor eingesetzten Rohr oder Schlauch ausgebildet sind.

5. Reaktor nach einem der Ansprüche 2 bis 4, dadurch gekennzeichnet, daß die Begasungs- und/oder Belüftungsöffnungen mit geringem Durchmesser, insbesondere im Bereich von <10 μm, vorzugsweise von 3 bis 6 μm, vorgesehen sind.

6. Reaktor nach einem der Ansprüche 2 bis 4, dadurch gekennzeichnet, daß als Begasungs- und/oder Belüftungsöffnungen Schlitze vorgesehen sind.

7. Reaktor nach einem der Ansprüche 2 bis 6, dadurch gekennzeichnet, daß das Verhältnis Reaktorrohrlänge zu hydraulischem Durchmesser mindestens 10:1 beträgt.

8. Reaktor nach einem der Ansprüche 2 bis 7, dadurch gekennzeichnet, daß im Abstand voneinander angeordnete, zumindest im wesentlichen im rechten Winkel zur Reaktorrohrachse verlaufende Einbauwände vorgesehen sind, die bei kreisförmigem Reaktorrohrquerschnitt die Form eines Kreisabschnittes aufweisen und im unteren Bereich des Reaktorrohrquerschnitts an der Reaktorrohrwand anliegen, so daß zwischen Reaktorrohrwand und Einbauwand jeweils ein sichelförmiger Spalt gebildet ist.

9. Reaktor nach Anspruch 8, dadurch gekennzeichnet, daß die Einbauwände im unteren Bereich einen, vorzugsweise keilförmigen, Ausschnitt aufweisen.

10. Reaktor nach einem der Ansprüche 8 oder 9, dadurch gekennzeichnet, daß die Einbauwände an einem in das Reaktorrohr einschiebbaren Träger befestigt sind.

11. Reaktor nach einem der Ansprüche 2 bis 10, dadurch gekennzeichnet, daß das Reaktorrohr in bekannter Weise als Mantelrohr aufgebaut ist, wobei der Mantelinnenraum vorzugsweise in Abschnitte unterteilt ist, die eine gezielte Temperaturführung über die Rohrlänge zulassen.

12. Reaktor nach einem der Ansprüche 2 bis 11, dadurch gekennzeichnet, daß das Reaktorrohr aus aneinandergeflanschten Abschnitten gebildet ist.

13. Reaktor nach einem der Ansprüche 2 bis 11, dadurch gekennzeichnet, daß in Abständen über die Reaktorlänge verteilte Abfuhr-und/oder Zufuhrstutzen für Medium und/oder Kontrollstutzen vorgesehen sind.

_Fig.1_

_Fig.2_

_Fig.3_

EP 0 377 429 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | BIOTECHNOLOGY & BIOENGINEERING, Band 26, Nr. 3, März 1984, Seiten 217-220, John Wiley & Sons, Inc., New York, US; G.K. CHOTANI et al.: "Immobilized cell cross-flow reactor" * Seite 217, Spalte 2, Zeile 35 - Seite 218, Spalte 2, Zeile 6; Figuren 1,2 * | 1-4,13 | C 12 M 1/04 B 01 J 10/00 C 02 F 3/12 |
| A | IDEM | 5-12 | |
| X | US-A-3 525 685 (R.N. EDWARDS) * Ansprüche 1,4; Fig.; Spalte 3, Zeile 51 - Spalte 4, Zeile 44 * | 1-5,7, 12,13 | |
| A | EP-A-0 211 109 (ARBIOS S.A.) * Ansprüche 1,2,3; Fig. * | 1,2,7,8 ,12,13 | |
| A | GB-A-1 540 142 (PHILLIPS PETROLEUM CIE) * Fig. * | 1,11 | |

RECHERCHIERTE SACHGEBIETE (Int. Cl.5)

C 12 M
C 02 F
B 01 J

Der vorliegende Recherchenbericht wurde für alle Patentansprüche erstellt

| Recherchenort | Abschlußdatum der Recherche | Prüfer |
|---|---|---|
| DEN HAAG | 23-04-1990 | COUCKE A.O.M. |